(19) Europäisches Patentamt
European Patent Office
Office européen des brevets

(11) **EP 4 471 798 A1**

(12) **EUROPEAN PATENT APPLICATION**

(43) Date of publication:
**04.12.2024 Bulletin 2024/49**

(21) Application number: **24167239.3**

(22) Date of filing: **28.03.2024**

(51) International Patent Classification (IPC):
**G16H 50/20** (2018.01)   **G16H 50/70** (2018.01)

(52) Cooperative Patent Classification (CPC):
**G16H 50/20; G16H 50/70;** G01N 21/3577;
G01N 21/552; G01N 2021/3595; G01N 2201/1296;
Y02A 90/10

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **02.06.2023   CN 202310648468**

(71) Applicant: **Sentry Medical Technology (Tianjin)
Co., Ltd
Tianjin 300192 (CN)**

(72) Inventors:
• **LI, Zhigang**
  **Tianjin, 300192 (CN)**
• **XU, Hanhui**
  **Tianjin, 300192 (CN)**
• **LI, Hong**
  **Tianjin, 300192 (CN)**

(74) Representative: **Dr. Gassner & Partner mbB
Wetterkreuz 3
91058 Erlangen (DE)**

(54) **DISEASE SCREENING MODEL BUILDING APPARATUS AND DISEASE SCREENING APPARATUS**

(57)    The present application relates to a disease screening model building apparatus, a disease screening apparatus, a device, and a medium. The disease screening model building apparatus comprises: a sample spectrum acquisition and data processing module; a plasma spectral digital biomarker determination module; a disease screening model building module, configured to build a disease screening model according to the training set and the validation set of the plasma samples and the spectral digital disease biomarker set by using a random forest of machine learning and a convolutional neural network of deep learning, and build a multi-model fused screening system; and a disease screening model testing module, configured to test the disease screening model and the fused screening system using the sample data in the test set to obtain a disease fusion screening model based on plasma membrane spectra. The present application can achieve high-throughput complex disease screening that is low-invasive, easy to operate, low-price, reliable in results, and environmentally friendly.

FIG. 1

EP 4 471 798 A1

## Description

### TECHNICAL FIELD

The present application relates to the technical field of intelligent medical detection and, in particular, to a disease screening model building apparatus and a disease screening apparatus.

### BACKGROUND ART

Currently, for more and more complex chronic diseases with multiple pathogenic factors, especially for complex diseases such as metabolic and neurodegenerative diseases, early screening and monitoring have become an urgent demand in the medical field.

However, among the existing medical screening and monitoring technologies, most of the screening and monitoring for complex diseases rely on scale screening, biochemical tests, imaging markers, etc., and have various shortcomings such as low time efficiency, high subjectivity, high invasiveness, environmental pollution, complex operation, and high price, failing to comply with the requirement of large-scale screening. For example, in the existing technologies, conventional methods for screening and detecting neurodegenerative diseases, such as Alzheimer's disease (AD), mainly include scale assessments, cerebrospinal fluid testing, and imaging examinations. The whole process of scale examination requires the participation of trained testers, and its use process is greatly affected by the education level and language understanding ability of test subjects and puts a high demand on the human resources of the testing provider. The cerebrospinal fluid biomarker based diagnostic method, having the shortcomings of complex operation, high invasiveness, patient's pain and the like, is not suitable for early detection of AD. Through the examinations of various brain functional imaging technologies such as fMRI, PET, and SPECT, the specific imaging manifestations of MCI/AD can be found and other potential diseases can also be excluded. However, due to the high testing cost, they cannot be routinely used in screening tests and mostly used for diagnosis of high-risk people.

Therefore, providing a high-throughput complex disease screening method and apparatus that are low-invasive, easy to operate, low in price, reliable in results, and environmentally friendly is an effective way to meet the current demand for large-scale screening of a complex disease with multiple pathogenic factors.

### SUMMARY

In order to achieve high-throughput complex disease screening that is low-invasive, easy to operate, low-price, reliable in results, and environmentally friendly, the present application provides a disease screening model building apparatus, a disease screening apparatus, a device, and a medium.

In a first aspect, the present application provides a disease screening model building apparatus, including:

a sample spectrum acquisition and data processing module, configured to acquire plasma samples from patients at different symptom stages of a disease and from healthy persons, and measure FTIR-ATR spectrum data of plasma membranes of the plasma samples to form a plasma spectrum sample set; and to divide spectrum samples in the plasma spectrum sample set according to different stages (early, middle and late states) of the disease relative to healthy persons to form a training set, a validation set and a test set for different levels of screening models for the disease relative to healthy persons, as well as for different courses of the disease relative to healthy persons;

a plasma spectral digital biomarker determination module, configured to determine a spectral digital disease biomarker set according to infrared FTIR-ATR spectra of the plasma membranes of the plasma samples; and

a disease screening model building module, configured to build a disease screening model according to the training set and the validation set of the plasma samples and the spectral digital disease biomarker set by using a random forest of machine learning and a convolutional neural network of deep learning, and build a multi-model fused screening system to obtain a disease fusion screening model based on plasma membrane spectra.

In the present application, especially by building the disease screening model according to the training set and the validation set of the plasma samples and the spectral digital disease biomarker set by using the random forest of machine learning and the convolutional neural network of deep learning and constructing the multi-model fused screening system, the high-throughput complex disease screening that is low-invasive, easy to operate, low-price, reliable in results, and environmentally friendly can be achieved. For example, it can achieve intelligent, rapid, highly sensitive and highly specific classification detection of complex diseases such as AD, depression, and various metabolic and neurodegenerative diseases.

In a second aspect, the present application provides a disease screening apparatus, including:
a disease screening model built by the disease screening model building apparatus described above.

## BRIEF DESCRIPTION OF THE DRAWINGS

FIG. 1 is a schematic module diagram of a disease screening model building apparatus in an embodiment of the present application.

FIG. 2 is a schematic diagram of a single sample plasma membrane prepared in an embodiment of the present application.

FIG. 3 is an exploded diagram of a sample loading platform used in preparation of plasma membranes in an embodiment of the present application.

FIG. 4 is a schematic diagram of a limiting and guide rail mechanism used for plasma membrane detection in an embodiment of the present application.

FIG. 5 is a schematic diagram of a limiting gasket in the limiting and guide rail mechanism.

FIG. 6 is a schematic diagram of a sample indenter and detection platform in the limiting and guide rail mechanism.

FIG. 7 is a schematic diagram of a sample plate guide rail structure in the limiting and guide rail mechanism.

FIG. 8 is a schematic diagram of plasma spectroscopy information of proteins, nucleic acids, lipids, hydrocarbons, glycogen, etc. obtained by using an FTIR spectrometer in an embodiment of the present application.

FIG. 9 is a schematic flowchart of a method for determining spectral digital biomarkers that can effectively screen diseases in an embodiment of the present application.

FIG. 10 is a schematic diagram of the correlation and comparison between traditional biomarkers and spectral digital biomarkers in an embodiment of the present application in disease screening (taking Alzheimer's disease as an example).

FIG. 11 is a schematic diagram of converting a spectral digital disease biomarker set into a two-dimensional GASF image in an embodiment of the present application.

FIG. 12 is a schematic workflow diagram of a GASF-CNN screening model in an embodiment of the present application.

FIG. 13 is a schematic structural diagram of a GASF-CNN screening model in an embodiment of the present application.

FIG. 14 is a schematic diagram of a building method of a fusion screening model in an embodiment of the present application.

FIG. 15 is a schematic structural diagram of a disease screening apparatus in an embodiment of the present application.

Listing of reference signs: 1. Sample spectrum acquisition and data processing module; 2. Plasma spectral digital biomarker determination module; 3. Disease screening model building module; 5. Limiting gasket; 6. Sample indenter and detection platform; 7. Sample plate guide rail structure.

## DETAILED DESCRIPTION

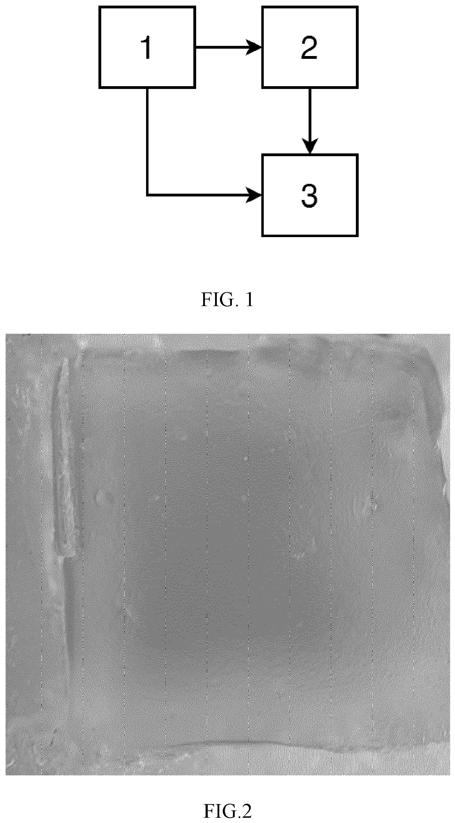

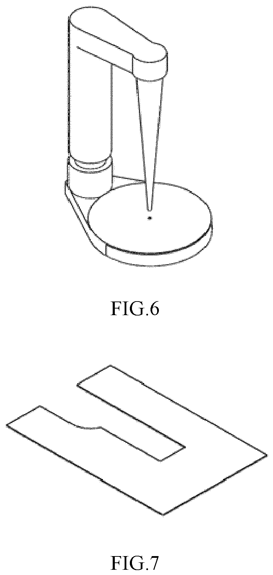

The present application will be further described in detail below with reference to FIGS. 1-15.

The present application discloses a disease screening model building apparatus. With reference to FIG. 1, a disease screening model building apparatus includes:

a sample spectrum acquisition and data processing module 1, configured to acquire plasma samples from patients at different symptom stages of a disease and from healthy persons and measure FTIR-ATR spectrum data of plasma membranes of the plasma samples to form a plasma spectrum sample set; and to distinguish spectrum samples in the plasma spectrum sample set according to different stages (early, middle and late stages) of the disease and healthy persons to form a training set, a validation set and a test set for different levels of screening models for the disease relative to healthy persons, as well as for different courses of the disease relative to healthy persons.

In an embodiment, the plasma membranes may be obtained by loading the plasma samples onto a plasma membrane sample plate in batches and drying the plasma samples. In this way, the interference of strong absorption spectrum peaks of water component on characteristic peaks of the test samples can be removed. A single sample plasma membrane is shown in FIG. 2. The plasma membrane sample plate here is made of an elastic silicone frame embedded with a carrier window. The infrared transmittance of the carrier window is greater than 90%. The plasma membrane sample plate may carry a QR code label with patient information. Specifically, the plasma membrane sample plate may be placed on a tray. There may be three columns and three rows of trays, carrying a total of nine independent plasma membrane sample plates, which is convenient for batch drying and independent measurement. In this way, the continuity of measurement can be ensured (that is, it can ensure that, after the spectrum of every plasma membrane is measured, the spectrum data of other plasma membranes can be measured), and the apparatus become small and lightweight. In other embodiments, each tray may also be provided with more or fewer independent plasma membrane sample plates. However, if more than nine independent plasma membrane sample plates are provided, in the process of measuring the spectrum data of one of the plasma membranes, it cannot be ensured that other plasma membranes are completely dried, so the continuity of measurement cannot be guaranteed. If less than nine independent plasma membrane sample plates are provided, it will reduce efficiency in large-scale measurement.

In an embodiment, the carrier window may be designed to have a surface area of (1.5-2 mm)*(1.5-2 mm), which is close to the surface area of an ATR test window (An exploded diagram of a sample loading platform is shown in FOG. 3. The surface area of the plasma membrane sample plate corresponds to the area of a sample piece in FOG. 3). In preparation of a plasma membrane, 0.5-1 uL of a plasma sample is dropwise added in the carrier window to form the membrane. As a result, a tiny amount of droplets can form a membrane evenly on such a small surface, avoiding the uneven membrane formation caused by a coffee ring effect in a large-area membrane formation process. The entire sample plate is loaded on an ATR test platform, which facilitates operation and improves test speed and stability.

In order to facilitate the operation, the carrier window may be arranged in a 10 mm* 10 mm sample assembly hole.

In addition, in a specific implementation, in order to ensure that a plasma membrane sample to be tested is in close contact with the ATR test window and has consistent elastic deformation and to improve the accuracy of spectroscopy, in the present application, the plasma membrane sample to be tested may also be positioned by using a limiting and guide rail mechanism so that the plasma membrane sample to be tested is in close contact with the ATR test window and has consistent elastic deformation, thereby achieving batch spectroscopy of plasma membranes.

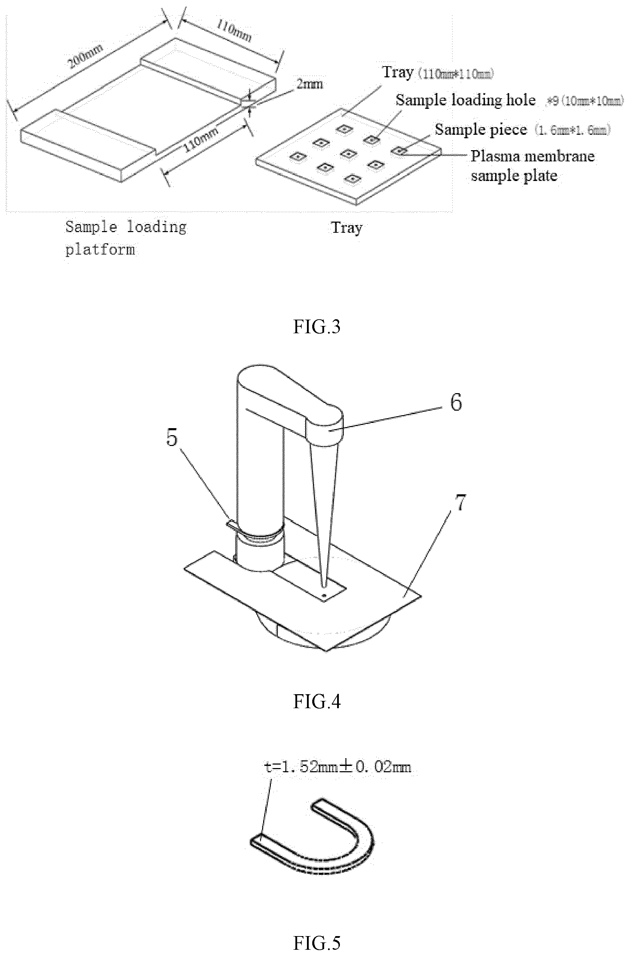

Specifically, by using a limiting gasket (the thickness t of the limiting gasket satisfies t = 1.52mm $\pm$ 0.02mm), the operating stroke of an indenter is limited, thereby avoiding excessive elastic deformation of or damage to the sample caused by an excessive force applied by the indenter and ensuring that the force acting on each sample plate is consistent. The membrane thickness will not deform beyond an allowable range due to excessive force applied by the indenter, thereby ensuring the reproducibility of signals and also ensuring that the test sample is basically consistent in membrane thickness and is in close contact with the ATR test platform. By using the sample plate guide rail structure, the samples can be quickly and accurately positioned to the ATR test window, improving test efficiency. The limiting and guide rail mechanism is shown in FIGS. 4-7.

A plasma spectral digital biomarker determination module 2, configured to determine a spectral digital disease biomarker set according to the FTIR-ATR spectrum (a digital biomarker preselection area determined by spectroscopy analysis is combined with a screening model by using a feature mining method) of the plasma membranes of the plasma samples;

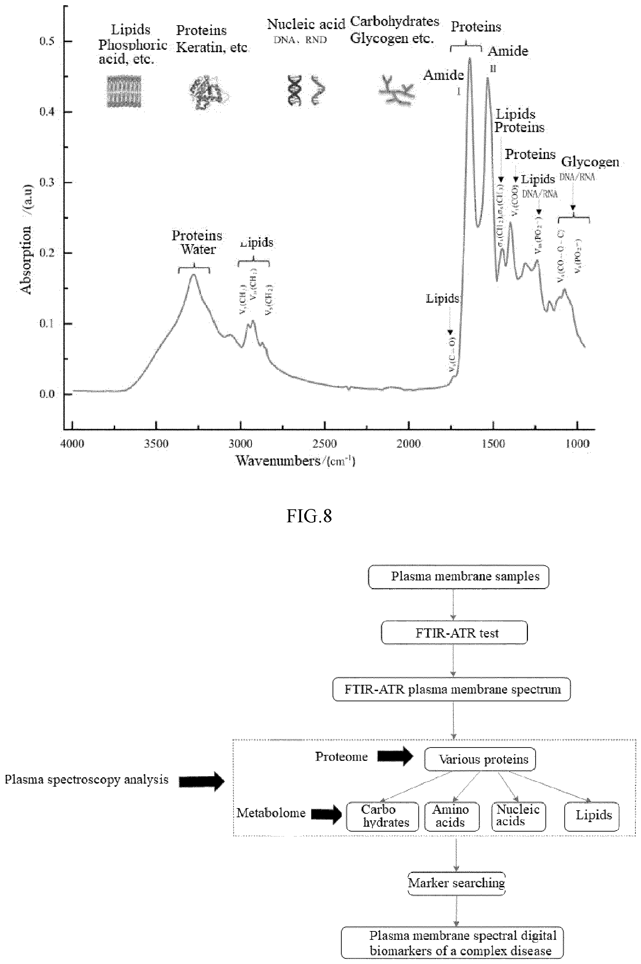

In an embodiment, in the process of measuring the FTIR-ATR spectrum data of the plasma membranes of the plasma samples, the optical absorption characteristics of the plasma membranes and the total reflection properties of the ATR accessory are used to acquire, based on the FTIR spectrometer, the omics information of proteins, nucleic acids, lipids, hydrocarbons, glycogen, etc in plasma spectra, as shown in FIG. 8. Based on the omics mapping relationship of proteins, lipids, nucleic acids, and carbohydrates in plasma spectra, within following ranges locked by the plasma spectroscopy, the statistical characteristics of the spectrum data in the training set are analyzed (using the two-sample KS (Kolmogorov-Smirnov) test or a filter method for feature selection (Relief) in machine learning), and then in combination with the validation set, spectral digital biomarkers capable of effectively screening the disease are determined (using a random forest screening model). As shown in FIG. 9, an area corresponding to the spectrum of proteins: 1700-1500 cm$^{-1}$; areas corresponding to the spectrum of lipids: 3050-2800 cm$^{-1}$, 1500-1350 cm$^{-1}$, 1745-1725 cm$^{-1}$ and 1270-1000 cm$^{-1}$; areas corresponding to the spectrum of nucleic acids: 1780-1550 cm$^{-1}$, 1550-1270 cm$^{-1}$, 1270-1000 cm$^{-1}$ and 1000-780 cm$^{-1}$; areas corresponding to the spectrum of hydrocarbons: 3600-3050 cm$^{-1}$, 3050 -2800 cm$^{-1}$, 1200-800 cm$^{-1}$ and 1500 -1200 cm$^{-1}$.

Specifically, the area 1700-1500 cm$^{-1}$ corresponding to the spectrum of proteins mainly involves amino acid side groups or peptide chains. The vibration modes of the peptide chains produce amide I and amide II bands, which are often used to study the secondary structure of proteins. This area includes 1700-1600 cm$^{-1}$ (the pathogenic protein-related molecular structure and functional group amide I band, mainly related to the stretching vibration of C=O); and 1600-1500 cm$^{-1}$ (the amide II band, mainly relates to the bending vibration of N-H bond). The areas corresponding to the spectrum of lipids: 3050-2800 cm$^{-1}$ (the asymmetric and symmetric stretching vibration area of pathogenic lipid-related molecular structures and functional groups -CH$_2$ and -CH$_3$); 1500-1350 cm$^{-1}$ (deformation vibration of -CH$_2$ and -CH$_3$ on fatty acyl chains); and 1745-1725 cm$^{-1}$ (symmetric stretching vibration of ester carbonyl bond C=O; and 1270-1000 cm$^{-1}$ (asymmetric and symmetric vibrations of pathogenic phospholipid-related molecular structures and functional group -PO2-. The spectrum of nucleic acids is distributed in four spectral areas: 1780-1550 cm$^{-1}$ (in-plane vibration of base double bond); 1550-1270 cm$^{-1}$ (coupling of base deformation vibration and sugar vibration); 1270-1000 cm$^{-1}$ (base deformation vibration); and 1000-780 cm$^{-1}$ (vibration of -PO2- and sugar-phosphate vibration). The spectrum of hydrocarbons is distributed in the following spectral areas: 3600-3050 cm$^{-1}$ (stretching vibration of O-H); 3050-2800 cm$^{-1}$ (stretching vibration of -CH$_3$ and -CH$_2$); 1200-800 cm$^{-1}$ (stretching vibration of C-O/C-C group); and 1500-1200 cm$^{-1}$ (deformation vibration of CH$_3$/CH$_2$ group).

The spectral digital AD biomarkers are mainly derived from the corresponding areas described above, and changes in molecular structures and functional groups in these areas often correspond to certain pathogenic factors and pathological characteristics.

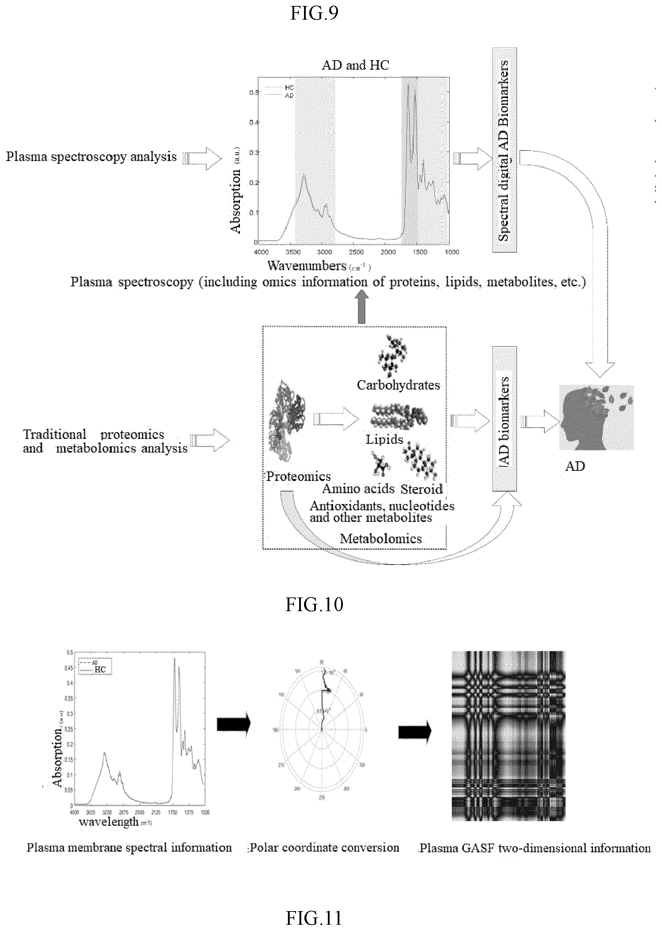

The correlation and comparison between traditional biomarkers and the spectral digital biomarkers in the present application in disease screening (taking AD as an example) are shown in FIG. 10.

In an embodiment, the spectral digital biomarkers are obtained by wavenumber importance ranking (two-sample KS test or the filter method for feature selection (Relief)) on the original spectrum, and the required characteristic wavenumbers are selected by model iteration testing as complex disease-related spectral digital biomarkers. The search process is as follow:

1) based on the plasma spectroscopy, determining the areas of proteins, lipids, nucleic acids and the like, locked by known infrared spectroscopy information, in FTIR-ATR plasma membranes, and searching for spectral digital disease biomarkers;

2) determining plasma spectrum intervals, wherein the observed absorption at a corresponding wavenumber $\upsilon$ of the spectrum is expressed as $\{X_{i_\upsilon}\}$, $i$ = 1 ... ... $n$, n is the total number of samples in the training set, the cumulative empirical distribution function of the absorption corresponding to the wavenumber is $F_\upsilon(\chi)$. Specifically, the cumulative empirical distribution functions of the plasma membrane spectrum of a patient with a certain complex disease and the plasma membrane spectrum of a healthy control group are $F_{\upsilon A}(\chi)$ and $F_{\upsilon H}(\chi)$, respectively;

3) based on the cumulative empirical distribution functions, defining a statistical variable that quantifies the maximum difference in the cumulative empirical distribution values of samples in the training set according to $D_{q,m}$ = max $(|F_{\upsilon A}(\chi) - F_{\upsilon H}(\chi)|)$, wherein the statistical variable is valued in an interval of [0, 1];

4) repeating the calculation to obtain the statistical variables $D_{q,m}$ corresponding to all wavenumbers and ranking all the statistical variables $D_{q,m}$ in a descending order to obtain the significance order of the wavenumbers, wherein the wavenumbers with high significance are suitable for priority as spectral biomarkers for distinguishing disease patients from healthy persons; and

5) based on significance ranking, conducting an iterative search for spectral digital biomarkers related to a complex disease by using a random forest classification model.

a disease screening model building module 3, configured to build a disease screening model according to the training set and the validation set of the plasma samples and the spectral digital disease biomarker set by using a random forest of machine learning and a convolutional neural network of deep learning, and build a multi-model fused screening system to obtain a disease fusion screening model based on plasma membrane spectra.

Specifically, the operation of building a disease screening model by using a random forest of machine learning and a convolutional neural network of deep learning and building a multi-model fused screening system to obtain a disease fusion screening model based on plasma membrane spectra includes:

S1, based on the training set and the validation set of one-dimensional plasma spectra, building a one-dimensional random forest disease screening model FTIR-RF by using a random forest classifier using the spectral digital disease biomarker set as input;

S2, converting the spectral digital disease biomarker set (equivalent to optimizing a one-dimensional signal that needs to be converted) into a two-dimensional GASF image for use as input, and building a two-dimensional random forest disease screening model GASF-RF by using a random forest classifier, wherein the two-dimensional GASF image can not only reflect spectral digital AD biomarkers, but also reflect the closeness of different spectral digital disease biomarkers, thereby improving the granularity of characteristic information, as shown in FIG 11;

specifically, the operation of building a two-dimensional random forest disease screening model GASF-RF by using a random forest classifier includes:

converting, by using the GASF, the one-dimensional data after feature selection into RGB color images for use as input data;

based on an image reading function, reading color image data after division of data sets, and converting image data format into a three-dimensional array composed of gray values in turn;

conducting a Flatten operation to convert the input data into a two-dimensional array format suitable for the input of the random forest classifier, and then implementing a binary classification task by using the random forest.

In an embodiment, the operation of converting the spectral digital disease biomarker set into a two-dimensional GASF image specifically includes: performing polar coordinate encoding on the infrared plasma spectrum data of the corresponding digital disease biomarkers as follows:

$$\begin{cases} \theta_i = arccos(A_i), & 0 \le A_i \le 1, \quad i = 1,2,3 \ldots \ldots 789 \\ r_i = \dfrac{N - i + 1}{N} \end{cases}$$

$i$ is the ranking number for the significance of a spectral digital biomarker (i.e., characteristic wavenumber); the maximum value of i is 789, and because a meaningful information area of mid-infrared combined plasma spectra is basically in the range of 4000-600 wavenumbers and the spectral resolution is selected as 4 cm⁻¹, there are 789 wavenumbers in this range; $A_i$ is the spectral absorption corresponding to a $i$-th spectral digital biomarker; $\theta_i$ is an encoded angle value corresponding to the $i$-th spectral digital biomarker; $N$ is the total number of spectral digital biomarkers; $r_i$ is an encoded radius corresponding to the $i$-th spectral digital biomarker.

The radius-weighted cosine function based GASF is generated as follows:

$$GASF = G(i,j) = \begin{bmatrix} cos(\theta_1 + \theta_1) & \cdots & cos(\theta_1 + \theta_j) \\ \vdots & \ddots & \vdots \\ cos(\theta_i + \theta_1) & \cdots & cos(\theta_i + \theta_j) \end{bmatrix}$$

All data fall within the unit circle of a polar coordinate system, the wavenumbers are encoded into different radii in the polar coordinate system, and the absorption is encoded into different angles in the polar coordinate system and irregularly extends outward in the polar coordinate system. Diagonal elements provide respective information of different spectral digital markers and the remaining elements provide related information between different spectral digital markers, which can not only reflect the spectral digital AD biomarkers, but also reflect the closeness of different spectral digital AD biomarkers. That is, the present application uses the GASF to convert the absorption of different spectral digital biomarkers into the cosine of the angular summation, and correlates the absorption information of all spectral digital biomarkers, thereby increasing the granularity of the information.

S3, based on the needs of the convolutional neural network of deep learning, constructing virtual samples by using an oversampling method such as SMTOE, Borderline SMOTE and ADASYN to expand the sample space of the training set; and based on the expanded new training set, converting the established spectral digital disease biomarker set into a two-dimensional GASF image for use as input information, and building a two-dimensional neural network disease screening model GASF-CNN by using a convolutional neural network (Resnet18, etc.) classifier of deep learning.

The wavenumbers of the one-dimensional data (i.e., the spectral digital disease biomarker set) mined by the filter method for feature selection are significantly representative, and at the same time, a large part of features are filtered to reduce redundant features and unimportant wavenumbers. Then, in a case of a small amount of selected features, it is not suitable to select a deep network architecture. Therefore, the inventors design a two-layer convolutional network that is more suitable for processing a small amount of features, which also reduces the amount of network parameters and speeds up calculations.

Specifically, the parameters of the neural network for the GASF-CNN screening model can be designed as follows: for a first convolution layer F1, the size of convolution kernels is set to (3, 3), the step size is set to 1, the number of convolution kernels is 32, the padding is set to 1, other parameters adopt default values, and data of three independent channels R, G and B are subjected to feature extraction respectively; for a second convolution layer F2, the size of convolution kernels is set to (5, 5), the step size is set to 2, and the number of convolution kernels is 128. A BN layer (for batch normalization) and a ReLu activation function are added in sequence between convolution layers. At the end of the convolutional neural network is a fully connected layer which converts an output two-dimensional feature map into a one-dimensional vector to achieve classification. The parameter of a first fully connected layer is set to 128 and the parameter of a second fully connected layer is set to 2, achieving binary classification. The ReLu function is used to activate between the two fully connected layers.

In the above neural network for the GASF-CNN screening model, the first convolution layer functions to perform feature extraction on the data of three independent channels and perform convolution operations by using different convolution kernels to extract different features. More convolution kernels are set in the second convolution layer to generate more features and make the receptive field of the model larger. In the meanwhile, by setting a large step size (the step size may be set to 2) and a large convolution kernel (the convolution kernel may be set to (5,5)), the image features can be further extracted.

In this embodiment, a larger convolution kernel and a larger step size are set in the second convolution layer to generate more features and make the receptive field of the model larger. In the meanwhile, by setting a large step size (the step size may be set to 2) and a large convolution kernel (the convolution kernel may be set to (5,5)), the image features can be further extracted. This arrangement is mainly for the following purposes: ① in the second layer, a larger kernel size is used to speed up the convergence rate; and ② a larger step size is used as an alternative to the max pooling layer to reduce data dimensionality. In this way, the screening speed of the model can be increased.

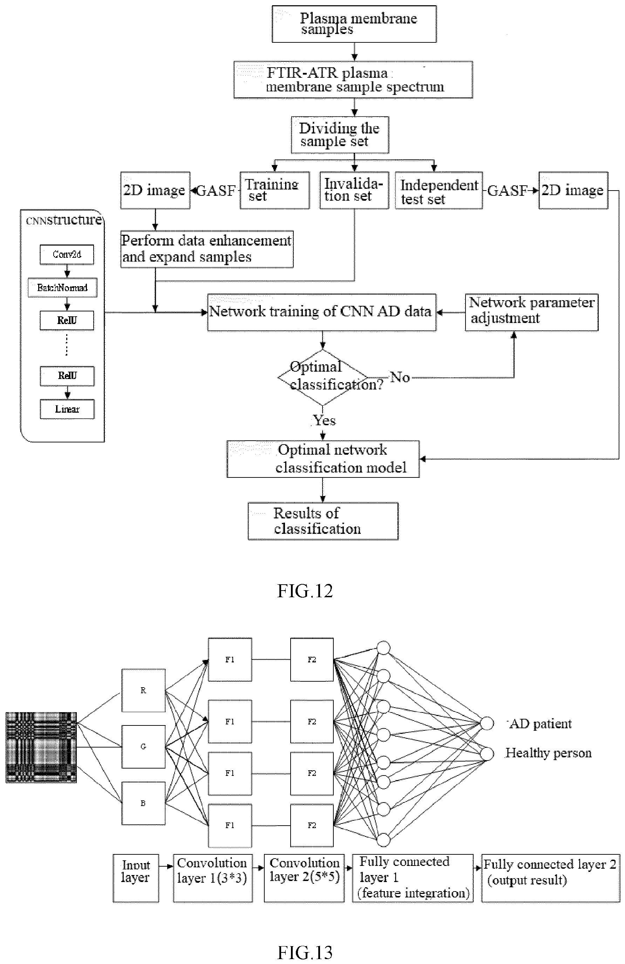

The building process flows of the above three models are similar. Taking the GASF-CNN screening model as an example, the building method flow of the GASF-CNN screening model is shown in FIG. 12, and the structure of the model is shown in FIG. 13. The building method of the GASF-CNN process flow includes:

> obtaining spectrum data of acquired plasma membrane samples by FTIR-ATR to form a plasma spectrum sample set;
> dividing spectrum samples in the plasma spectrum sample set according to different stages of the disease relative to healthy persons to form a training set, a validation set and a test set for different levels of screening models for different courses of the disease relative to healthy persons;
> performing data enhancement on samples in the training set and adding data features to obtain an expanded new training set;
> using the established spectral digital AD biomarker set to perform GASF transformation on the spectrum data of the training set, the validation set and the test set respectively to form a two-dimensional image data set containing spectral information and using the two-dimensional image data set as input information; and
> performing model training by using a convolutional neural network of deep learning, searching for optimal model parameters by setting conditional loop iteration, and then evaluating the generalization ability of the model by using samples in the test set, and finally outputting a final screening result.

In the above method, the one-dimensional spectrum data is converted into a two-dimensional image by using the GASF technology, and by using the convolutional neural network, the optimal features are automatically extracted for classification, thereby effectively capturing the characteristic information of Alzheimer's disease and avoiding complex preprocessing and feature extraction processes. Therefore, the method has the advantages of high efficiency, simplicity, and good robustness.

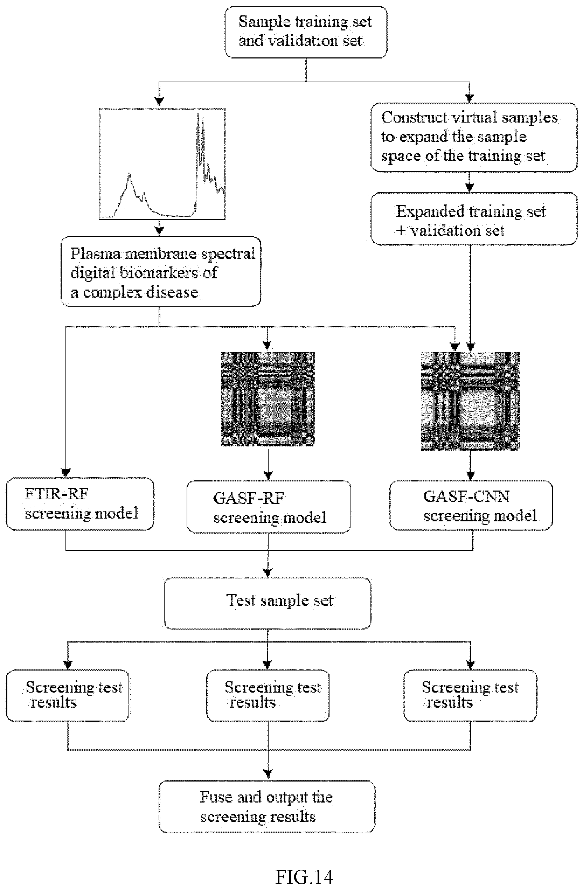

S4, based on the test set, testing the three models, wherein for a same test sample, after each model outputs a corresponding screening result, the output results of the three models are fused by voting (i.e., the principle of minority obeying the majority) to output a final screening result, thus obtaining a disease fusion screening model based on the plasma membrane spectrum digital disease biomarker set to complete the screening and auxiliary diagnosis of different stages of a complex disease relative to healthy persons. As shown in FIG. 14,

In an embodiment, the following method can be used to construct a virtual sample, expand the sample space of the training set to obtain an expanded new training set:

> selecting a sample $S_i$ from plasma spectrum minority class samples;
> randomly selecting N samples $S_{zi}$ from K nearest neighbors of $S_i$ at a sampling rate N;
> randomly synthesizing new minority class plasma spectrum samples between $S_i$ and $S_{zi}$ in sequence as follows:

$$S_n = S_i + \beta(S_{zi} - S_i), \ \beta \in [0,1]$$

> and is a random number;
> if more than half of samples surrounding a new sample are minority class samples, considering the new sample as a valid virtual sample;
> if more than half but not all of samples surrounding the new sample are majority class samples, considering the new sample as a sample on the boundary; during synthesis, randomly selecting a minority class sample are from the K nearest neighbors as $S_{zi}$, and then performing synthesis as above; and
> if a new sample is surrounded by majority class samples, considering the new sample as noise that should be discarded, and then discarding the virtual sample.

In other embodiments, virtual samples may also be constructed using generation technology based on K nearest neighbor algorithm, generation technology based on data perturbation, generation technology based on interval kernel density estimation, etc.

Those skilled in the art can clearly understand that for the convenience and simplicity of description, only the division of the above functional modules is described as an example. In practical applications, the foregoing functions may be allocated for and completed by different functional modules as needed. That is, the internal structure of the apparatus is divided into different functional modules to accomplish all or part of the functions described above.

Embodiments of the present application further disclose a disease screening apparatus. A disease screening apparatus includes a disease screening model built by the disease screening model building apparatus according to any one of aspects described above.

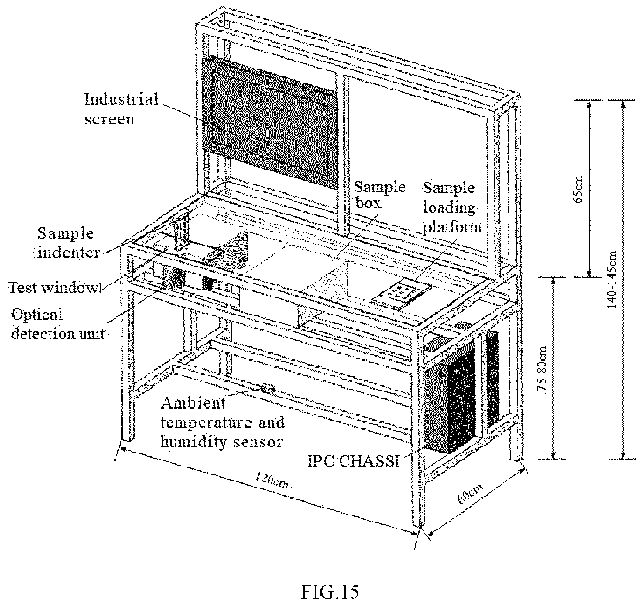

In specific implementation, the disease screening apparatus may be a disease screening platform as shown in FIG. 15, and may also include an optical detection unit, a sample loading platform, a sample box, an industrial personal computer (i.e., an IPC CHASSI, equipped with the disease screening model built by the disease screening model building apparatus), an ambient temperature and humidity sensor, an industrial screen, a limiting and guide rail mechanism, and the like.

**Experimental example**

203 AD samples (68 samples of early symptoms, 117 samples of middle symptoms, and 18 samples of late symptoms) are provided. The sample spectrum acquisition and data processing module first acquire a specified number of plasma samples from Alzheimer's disease patients at different symptom stages and from healthy people. Nine independent plasma membrane sample plates are placed on the sample loading platform each time to carry 0.5-1 uL-scale plasma droplets in batches and the plasma droplets are dried into membranes. The plasma membrane sample plate is a carrier for membrane formation of the plasma samples. The plasma membrane sample plate is composed of an elastic silicone frame embedded with a carrier window made of an infrared-absorbing and low-absorbing material.

Based on the optical absorption properties of the plasma membrane and the total reflection properties of an ATR accessory, the ATR test accessory of the FTIR spectrometer obtains plasma spectromic signals containing information of proteins, lipids, nucleic acids, hydrocarbons, etc. Each sample is scanned and measured 10 times, and the average spectrum is taken as the FTIR-ATR spectrum measurement value to obtain the plasma FTIR-ATR spectrum data of patients with Alzheimer's disease. The average spectrum and related omics information of patients with Alzheimer's disease in this example are shown in FIG. 12.

During the process of testing plasma membrane samples, the operating stroke of an indenter is limited by a limiting means, thereby avoiding excessive elastic deformation of or damage to the sample caused by an excessive force applied by the indenter and ensuring that the force acting on each sample plate is consistent. The membrane thickness will not deform beyond an allowable range due to the force applied by the indenter, thereby ensuring that the test sample is basically consistent in membrane thickness and is in close contact with the ATR test platform and guaranteeing the quality of its spectral signal and the reproducibility of the signal. The guide rail mechanism is configured to quickly and accurately position the sample to the ATR test window to improve test efficiency.

The plasma membrane spectrum sample set is divided according to a sample number ratio of 7:1.5:1.5 according to the early, middle and late stages of Alzheimer's disease relative to the spectrum samples of healthy persons, forming training sets, validation sets, and test sets for screening models for Alzheimer's disease, Alzheimer's disease of different disease courses, and healthy persons at different levels and scales;

The significance of the spectral characteristic wavenumbers in the training set is analyzed by using a feature mining method of the two-sample KS test or the filter method for feature selection (Relief) in machine learning. Then, based on the training set and the validation set, in combination with the plasma spectroscopy method, the structural and biochemical information of proteins, lipids, nucleic acids and small molecule metabolites in the plasma membranes are analyzed, and the random forest classification model is used to search for AD-related spectral digital biomarkers. Then, based on the GASF, the one-dimensional spectrum containing omics information of proteins, lipids and metabolites is converted into two-dimensional image information to improve the granularity of characteristic information;

Aiming at the heterogeneity between different courses of AD, the one-dimensional information of the plasma membrane spectrum of the training set and the converted GASF two-dimensional image information are used as input information to build an FTIR-RF screening model, a GASF-RF screening model and a GASF-CNN screening model based on random forest (RF) and a self-built convolutional neural network (CNN), respectively. Parameter settings of the random forest screening model: the number of decision trees ntree is 200, the number of randomly sampled variables mtry is 8 during the construction of a decision tree branch, and changes are made continuously step by step according to the results to find the most suitable model parameters. Finally, a fusion screening model suitable for different stages of a complex disease is built from the above three models to complete the screening and auxiliary diagnosis of a certain complex disease at different stages relative to healthy persons.

More specifically, the neural network for the GASF-CNN screening model is designed as follows: for a first convolution layer F1, the size of convolution kernels is set to (3, 3), the step size is set to 1, the number of convolution kernels is 32, the padding is set to 1, other parameters adopt default values, and three independent channel data of R, G and B are subjected to feature extraction respectively; for a second convolution layer F2, the size of convolution kernels is set to (3, 3), the step size is set to 2, the step size is set to 1, the number of convolution kernels is 64, and the padding is set to 1. A BN layer (for batch normalization) and a ReLu activation function are added in sequence between convolution layers. At the end of the convolutional neural network is a fully connected layer which converts an output two-dimensional feature map into a one-dimensional vector to achieve classification. The parameter of a first fully connected layer is set to 128 and the parameter of a second fully connected layer is set to 2, achieving binary classification. The ReLu function is used to activate between the two fully connected layers.

Evaluation Indicators of the complex disease screening and auxiliary diagnosis model based on plasma spectrum constructed by the present application may be defined by a confusion matrix, and the confusion matrix H is expressed as:

$$H = \begin{bmatrix} TP & FP \\ FN & TN \end{bmatrix}$$

where, TP: determined to be positive, but actually positive, that is, true positive; FN: determined to be negative, but actually positive, that is, false negative; FP: determined to be positive, but actually negative, that is, false positive; TN: determined to be negative, actually negative, that is, true negative;

Indicators in the field of disease detection include sensitivity and specificity. The sensitivity refers to the probability of no missed diagnosis during screening and diagnosis, and the specificity refers to the probability of no misdiagnosis during screening and diagnosis. The sensitivity and the specificity are expressed as:

$$sensitivity = \frac{TP}{TP + FN} \times 100\%$$

$$specificity = \frac{TN}{FP + TN} \times 100\%$$

In the evaluation of the screening and diagnosis model of Alzheimer's disease, the two evaluation indicators of sensitivity and specificity are used to objectively evaluate the application performance of the model. The results of this example are as follows:

| Method | | Validation set | | Test set | |
|---|---|---|---|---|---|
| | | Sensi tivity | Speci ficity | Sensi tivity | Speci ficity |
| Early-s tage AD | FTIR-RF | 90% | 94.74 % | 90% | 84.21 % |
| | GASF-RF | 80% | 94.74 % | 80% | 89.47 % |
| | GASF-CN N | 90% | 89.5 % | 90% | 84.21 % |
| | Fusion screening model of the present application | 100% | 94.74 % | 90% | 94.74 % |
| Mid-st age AD | FTIR-RF | 91.67 % | 83.33 % | 90.91 % | 83.33 % |
| | GASF-RF | 83.33 % | 83.33 % | 90.91 % | 83.33 % |
| | GASF-CN N | 83.33 % | 83.33 % | 81.81 % | 83.33 % |
| | Fusion screening model of the present application | 100% | 83.33 % | 90.91 % | 100% |
| Late-st age AD | FTIR-RF | 100% | 100% | 100 % | 100% |
| | GASF-RF | 100% | 100% | 100 % | 100% |
| | GASF-CN N | 100% | 100% | 100 % | 100% |
| | Fusion screening model of the present application | 100% | 100% | 100 % | 100% |

It can be seen that, compared with the individual FTIR-RF model, GASF-RF model and GASF-CNN, in the screening tests of the disease at easy and middle stages (mainly focusing on the test set ), when the fusion screening model of the present application for disease screening has higher specificity while ensuring sensitivity.

**Claims**

1. A disease screening model building apparatus, **characterized by** comprising:

a sample spectrum acquisition and data processing module (1), configured to acquire plasma samples from patients at different symptom stages of a disease and from healthy persons and measure FTIR-ATR spectrum data of plasma membranes of the plasma samples to form a plasma spectrum sample set; and to classify spectrum samples in the plasma spectrum sample set according to different stages, including early, middle and late stages, of the disease and healthy persons to form a training set, a validation set and a test set for different levels of screening models for the disease relative to healthy persons and for different courses of the disease relative to healthy persons;
a plasma spectral digital biomarker determination module (2), configured to determine a spectral digital disease biomarker set according to infrared FTIR-ATR spectra of the plasma membranes of the plasma samples; and
a disease screening model building module (3), configured to build a disease screening model according to the training set and the validation set of the plasma samples and the spectral digital disease biomarker set by using a random forest of machine learning and a convolutional neural network of deep learning, and build a multi-model fused screening system to obtain a disease fusion screening model based on plasma membrane spectra.

2. The disease screening model building apparatus according to claim 1, **characterized in that**, the plasma membranes are obtained by loading the plasma samples onto a plasma membrane sample plate and drying the plasma samples, wherein the plasma membrane sample plate is made of an elastic silicone frame embedded with a carrier window, and an infrared transmittance of the carrier window is greater than 90%

3. The disease screening model building apparatus according to claim 2, **characterized in that**, the carrier window has a surface area of (1.5-2 mm)*(1.5-2 mm), and in preparation of a plasma membrane, 0.5-1 uL of a plasma sample is dropwise added to the carrier window to form the plasma membrane.

4. The disease screening model building apparatus according to claim 1, **characterized in that**, based on an omics mapping relationship of proteins, lipids, nucleic acids, and carbohydrates in plasma spectra, statistical characteristics of the spectrum data in the training set are analyzed, and spectral digital biomarkers capable of effectively screening the disease are determined in combination with the validation set, within the following ranges determined by the plasma spectroscopy: an area corresponding to the spectrum of proteins: 1700-1500 $cm^{-1}$; areas corresponding to the spectrum of lipids: 3050-2800 $cm^{-1}$, 1500-1350 $cm^{-1}$, 1745-1725 $cm^{-1}$ and 1270-1000 $cm^{-1}$; areas corresponding to the spectrum of nucleic acids: 1780-1550 $cm^{-1}$, 1550-1270 $cm^{-1}$, 1270-1000 $cm^{-1}$ and 1000-780 $cm^{-1}$; areas corresponding to the spectrum of hydrocarbons: 3600-3050 $cm^{-1}$, 3050 -2800 $cm^{-1}$, 1200-800 $cm^{-1}$ and 1500-1200 $cm^{-1}$.

5. The disease screening model building apparatus according to claim 1, **characterized in that**, a step of building a disease screening model by using a random forest of machine learning and a convolutional neural network of deep learning and building a multi-model fused screening system to obtain a disease fusion screening model based on plasma membrane spectra comprises:

based on the training set and the validation set of one-dimensional plasma spectra, building a one-dimensional random forest disease screening model FTIR-RF by using a random forest classifier using the spectral digital disease biomarker set as an input;
converting the spectral digital disease biomarker set into a two-dimensional GASF (Gramian Angular Summation Field) image for use as input, and building a two-dimensional random forest disease screening model GASF-RF by using a random forest classifier;
constructing virtual samples to expand the sample space of the training set; and based on the expanded new training set, converting the established spectral digital disease biomarker set into a two-dimensional GASF image for use as input information, and building a two-dimensional neural network disease screening model GASF-CNN by using a convolutional neural network classifier of deep learning; and
based on the test set, performing testing on the one-dimensional random forest disease screening model FTIR-RF, the two-dimensional random forest disease screening model GASF-RF and the two-dimensional neural network disease screening model GASF-CNN, wherein for a same test sample, after each model outputs a corresponding screening result, the output results of the three models are fused to output a final screening result, thus obtaining a disease fusion screening model based on the plasma membrane spectrum digital disease biomarker set to complete the screening and auxiliary diagnosis of different stages of the disease relative to healthy persons.

**6.** The disease screening model building apparatus according to claim 5, **characterized in that**, a step of converting the spectral digital disease biomarker set into a two-dimensional GASF image comprises: performing polar coordinate encoding on infrared plasma spectrum data of corresponding digital disease biomarkers as follows:

$$\begin{cases} \theta_i = arccos(A_i), & 0 \le A_i \le 1, \quad i = 1,2,3 \dots \dots 789 \\ r_i = \dfrac{N - i + 1}{N} \end{cases}$$

where, $i$ is a ranking number for significance of a spectral digital biomarker; $A_i$ is the spectral absorption value corresponding to an $i$-th spectral digital biomarker; $\theta_i$ is an encoded angle value corresponding to the $i$-th spectral digital biomarker; $N$ is the total number of spectral digital biomarkers; $r_i$ is an encoded radius corresponding to the $i$-th spectral digital biomarker.

**7.** The disease screening model building apparatus according to claim 5, **characterized in that**, a step of constructing a virtual sample to expand the sample space of the training set to obtain an expanded new training set comprises:

selecting a sample $S_i$ from plasma spectrum minority class samples;
randomly selecting N samples $S_{zi}$ from K nearest neighbors of $S_i$ at a sampling rate N;
randomly synthesizing new minority class plasma spectrum samples between $S_i$ and $S_{zi}$ in sequence as follows:

$$S_n = S_i + \beta(S_{zi} - S_i), \quad \beta \in [0,1]$$

and is a random number;
when more than half of samples surrounding a new sample are minority class samples, considering the new sample as a valid virtual sample;
when more than half but not all of samples surrounding the new sample are majority class samples, considering the new sample as a sample on the boundary; and during synthesis, randomly selecting a minority class sample from the K nearest neighbors as $S_{zi}$, and then performing synthesis as above; and
when a new sample is surrounded by majority class samples, considering the new sample as noise to be discarded, and discarding the virtual sample.

**8.** A disease screening apparatus, **characterized by** comprising a disease screening model built by the disease screening model building apparatus according to any one of claims 1-7.

FIG. 1

FIG.2

200mm

110mm

2mm

110mm

Tray (110mm*110mm)

Sample loading hole *9 (10mm*10mm)

Sample piece (1.6mm*1.6mm)

Plasma membrane sample plate

Sample loading platform

Tray

FIG.3

5

6

7

FIG.4

t=1.52mm±0.02mm

FIG.5

FIG.6

FIG.7

FIG.8

FIG.9

AD and HC

Plasma spectroscopy analysis

Spectral digital AD Biomarkers

Plasma spectroscopy (including omics information of proteins, lipids, metabolites, etc.)

Traditional proteomics and metabolomics analysis

Carbohydrates

Lipids

Proteomics

Amino acids Steroid

Antioxidants, nucleotides and other metabolites

Metabolomics

IAD biomarkers

AD

FIG.10

Plasma membrane spectral information    ;Polar coordinate conversion    .Plasma GASF two-dimensional information

FIG.11

FIG.12

FIG.13

FIG.14

FIG.15

Europäisches
Patentamt

European
Patent Office

Office européen
des brevets

# EUROPEAN SEARCH REPORT

**Application Number**

EP 24 16 7239

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 115 274 100 A (PUNING MEDICAL TECH TIANJIN CO LTD) 1 November 2022 (2022-11-01) * paragraphs [0010], [0021], [0072] - [0074], [0084], [0092], [0093], [0094] * | 1-8 | INV. G16H50/20 G16H50/70 |
| X | CN 112 444 500 B (UNIV NORTHEASTERN QINHUANGDAO) 24 June 2022 (2022-06-24) * whole document, in particular claim 1 * | 1-8 | |
| A | PARASKEVAIDI MARIA ET AL: "Blood-based near-infrared spectroscopy for the rapid low-cost detection of Alzheimer's disease", ANALYST, vol. 143, no. 24, 1 January 2018 (2018-01-01), pages 5959-5964, XP093204212, UK ISSN: 0003-2654, DOI: 10.1039/C8AN01205A * the whole document * | 1-8 | |

**TECHNICAL FIELDS SEARCHED (IPC)**

G16H
G01N

The present search report has been drawn up for all claims

| Place of search | Date of completion of the search | Examiner |
|---|---|---|
| Munich | 11 September 2024 | Mühlbauer, Max |

EPO FORM 1503 03.82 (P04C01)

## ANNEX TO THE EUROPEAN SEARCH REPORT
## ON EUROPEAN PATENT APPLICATION NO.

EP 24 16 7239

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2024

| Patent document cited in search report | | Publication date | Patent family member(s) | Publication date |
|---|---|---|---|---|
| CN 115274100 | A | 01-11-2022 | NONE | |
| CN 112444500 | B | 24-06-2022 | NONE | |

EPO FORM P0459

For more details about this annex : see Official Journal of the European Patent Office, No. 12/82